# EUROPÄISCHE PATENTSCHRIFT

(11) **EP 3 460 447 B1**
(45) Veröffentlichungstag und Bekanntmachung des Hinweises auf die Patenterteilung: **07.06.2023**
(21) Anmeldenummer: 17192853.4
(22) Anmeldetag: 25.09.2017
(51) Int. Cl.: G01N 3/12, G01N 33/38

(54) **PRÜFANORDNUNG FÜR BRUCHSPANNUNG**
TEST ASSEMBLY FOR FRACTURE STRESS
DISPOSITIF D'ESSAI DE TENSION DE FRACTURE

(43) Veröffentlichungstag der Anmeldung: 27.03.2019
(73) Patentinhaber: Lerch, Markus, 44263 Dortmund (DE)
(72) Erfinder: Lerch, Markus, 44263 Dortmund (DE)
(74) Vertreter: Kalkoff & Partner Patentanwälte mbB

(56) Entgegenhaltungen:
- DE-A1- 4 138 416
- DE-B3-102015 108 376

## Beschreibung

Die Erfindung betrifft eine Prüfanordnung zum Messen der Bruchspannung sowie ein Prüfmittel.

Prüfanordnungen zum Messen der Bruchspannung, insbesondere zum zerstörenden Messen, sind z. B. aus der DE 10 2015 108376 B3 und der DE 41 38 416 A1 bekannt. Es werden z. B. Vorrichtungen zur Prüfung der Zugfestigkeit eingesetzt, die aufwändig und teuer sind. Sie eigenen sich nicht, um z. B. zur Qualitätskontrolle begleitend zu einer Produktion von z. B. keramischen Gegenständen eingesetzt zu werden.

Es besteht Bedarf nach einer einfachen Prüfanordnung und einem einfachen Verfahren zum Prüfen bzw. Messen der Bruchspannung, die insbesondere zur Qualitätskontrolle eingesetzt werden können.

Die Erfindung löst diese Aufgabe mit einer Prüfanordnung nach Anspruch 1 und mit einem Verfahren nach Anspruch 10.

Die erfindungsgemäße Prüfanordnung zum Erfassen der Bruchspannung weist einen Antrieb auf, der kraft- oder drehzahlgesteuert ist, und der nach einer ersten Alternative mit einer Kolben-Zylinder-Anordnung und nach einer zweiten Alternative mit einer Pumpe verbunden ist, wobei die Kolben-Zylinder-Anordnung oder die Pumpe am ersten Ende einer Druckleitung angeschlossen ist und wobei das zweite Ende der Druckleitung einen Anschluss für ein Prüfmittel aufweist. Der Antrieb weist meist eine Leistung von ca. 50 bis 1500 W auf.

Der Antrieb ist vorzugsweise ein elektrischer Antrieb, der entweder kraftgesteuert ist, z. B. mittels einer Strom- oder Leistungssteuerung oder einer Drehmomentregelung, oder ein Antrieb, der drehzahlgesteuert ist. Auf diese Weise wird gewährleistet, dass ein gleichmäßiger Druckaufbau in der Druckleitung erzeugt wird.

Die Kolben-Zylinder-Anordnung weist eine elektrische Kolben-Zylinder-Anordnung auf, die mit dem Antrieb in Verbindung steht und sie weist erfindungsgemäß eine hydraulische Kolben-Zylinder-Anordnung auf, deren Kolben in fester Verbindung mit dem Kolben der elektrischen Kolben-Zylinder-Anordnung steht. Die hydraulische Kolben-Zylinder-Anordnung steht weiter mit einer Druckleitung in Strömungsverbindung, so dass sie über den Antrieb und die elektrische Kolben-Zylinder-Anordnung Druck auf das in der Druckleitung befindliche Fluid aufbringen kann. Der hydraulische Kolben weist typisch einen Hub von 100 mm auf. Die Kolben-Zylinder-Anordnung ist für jede Art von Antrieb geeignet, unabhängig davon, ob der Antrieb kraft- oder drehzahlgesteuert ist.

Alternativ ist eine Pumpe zwischen dem Antrieb und der Druckleitung eingesetzt. Die Pumpe ist insbesondere bei drehzahlgesteuertem Antrieb gut einsetzbar.

Die Druckleitung ist zum Leiten eines Fluids, insbesondere einer unkomprimierbaren Flüssigkeit, typisch Wasser oder Öl, ausgelegt. Sie hält, ausgehend vom Atmosphärendruck, einem Druck von bis zu 200 bar, insbesondere von bis zu 100 bar, vorzugsweise von bis zu 50 bar, mindestens bis zu 20 bar stand. Die Druckleitung ist meist aus Kunststoff oder Metall und weist einen Innendurchmesser von ca. 4-5 mm auf. Die Druckleitung steht mit der Pumpe oder der Kolben-Zylinder-Anordnung in Strömungsverbindung. Die Druckleitung ist mit dem Prüfmittel bzw. mit der zu prüfenden Probe verbunden. Nach einer vorteilhaften Ausführung weist die Druckleitung mindestens ein, insbesondere zwei Ventile auf, bevorzugt sind dies Magnetventile. Bevorzugt weist die Druckleitung ein Auslauf- und ein Einlaufventil auf, mit dem das Fluid in die Druckleitung eingefüllt und aus der Druckleitung abgelassen wird. Das Fluid strömt vom Einlaufventil zum Auslaufventil. Damit ist die Strömungsrichtung festgelegt. Die im Zusammenhang mit der Beschreibung der Erfindung verwendeten Begriffe "stromauf und "stromab" beziehen sich auf die Strömungsrichtung. Während der Prüfung einer Probe ist die Druckleitung eine geschlossene Anordnung, deren Druck durch Betätigen der Kolben-Zylinder-Anordnung bzw. der Pumpe während des Messvorgangs gesteigert wird.

Insbesondere, wenn eine hydraulische Kolben-Zylinder-Anordnung an die Druckleitung angeschlossen ist, ist nach einer vorteilhaften Ausführung ein Sperrventil benachbart zu dieser Kolben-Zylinder-Anordnung auf der dem Einlaufventil abgewandten Seite der Kolben-Zylinder-Anordnung in die Druckleitung eingesetzt. Das Sperrventil dient beim Fluten der Druckleitung dazu, während des Anlaufens bzw. In-Betrieb-Nehmens der hydraulischen Kolben-Zylinder-Anordnung ein Leersaugen der Druckleitung zu verhindern. Nach dem Fluten der Druckleitung und in allen anderen Betriebszuständen ist das Sperrventil geöffnet.

Wenn bekannt ist, dass das zu messende Werkstück nur geringem Prüfdruck standhält, so, wie es z. B. bei der Messung dünner keramischer Schichten der Fall ist, dann wird die erfindungsgemäße Prüfanordnung z. B. mit einem Druck von nur zwei bar betrieben. Wenn jedoch Werkstücke gemessen werden, die einem höheren Prüfdruck standhalten, dann wird die erfindungsgemäße Prüfanordnung mit einem Druck von z. B. 20 bar betrieben. Entsprechend weist die Prüfanordnung bzw. deren Druckleitung eine Verzweigung mit mindestens einer ersten und einer zweiten parallel angeordneten Zweigleitung auf. Die erste Zweigleitung weist einen Drucktransmitter für einen niedrigeren Messdruck von z. B. 2 bar auf, die zweite Zweigleitung weist einen Drucktransmitter für einen höheren Messdruck von z. B. 20 bar auf. Jede Zweigleitung kann durch ein Zweigleitungsventil, das in Strömungsrichtung vor dem Drucktransmitter angeordnet ist, geschlossen oder geöffnet werden. Ist ein Zweigleitungsventil geschlossen, dann strömt das gesamte Fluid durch die jeweils andere Zweigleitung. Zusätzlich ist in der ersten Zweigleitung, die für das Messen des niedrigeren Drucks ausgelegt ist, ein zweites Zweigleitungsventil in Strömungsrichtung hinter dem Drucktransmitter angeordnet. Auf diese Weise wird der empfindliche Drucktransmitter in der ersten Zweigleitung der Verzweigung abgeschottet, wenn die Prüfanordnung bei höherem Druck betrieben wird. Weitere Drucktransmitter können bei Bedarf nach der vorbeschriebenen Konstruktion in der erfindungsgemäßen Prüfanordnung eingesetzt werden. Die Drucktransmitter messen mit einer Genauigkeit von bis zu 5 mbar, bevorzugt von bis zu 1 mbar, je nach dem Auflösungsvermögen der Drucktransmitter.

Nach einem weiteren Aspekt der Erfindung, die das Prüfen bei erhöhtem Druck wesentlich vereinfacht oder die bei einer Prüfanordnung, die mit einem sehr geringen Flüssigkeitsvolumen in der Druckleitung arbeitet, eingesetzt wird, wird nachfolgend geschildert: Bei einem drehzahlgesteuerten Antrieb der Prüfanordnung ist erfindungsgemäß eine zweite Verzweigung der Druckleitung vorgesehen. In dieser Kapillarleitung ist eine Querschnittsverengung angebracht, die parallel zur Druckleitung verläuft. Die Querschnittsverengung ist erfindungsgemäß in Strömungsrichtung nach der zu prüfenden Probe angebracht. Die Querschnittsverengung ist vorteilhaft als Kapillare ausgeführt, die ein erstes, mit der Verzweigung der Druckleitung verbundenes Ende aufweist und ein zweites Ende, aus dem die Flüssigkeit in eine drucklose Auslaufleitung strömt. Eine Kapillare kann z. B. einen inneren Durchmesser von 0,5 mm und eine Länge von 1 m aufweisen.

Entsprechend des Kontinuitätsgesetzes (Bernoulli-Gleichung) wird stromaufwärts der Kapillare proportional zur Strömungsgeschwindigkeit der statische Druck in der Druckleitung, also auf der Seite des größeren Querschnitts, erhöht. Bei dieser Anordnung wird der höhere Prüfdruck wesentlich schneller erreicht als bei der einfachen Ausführung der Prüfanordnung.

In die erfindungsgemäße Prüfanordnung, die es ermöglicht, die Querschnittsverengung optional in die Druckleitung einzuschalten, ist ein zweites Sperrventil stromab der Verzweigung zur Kapillare in die Druckleitung eingesetzt. Wenn die Kapillare genutzt werden soll, wird die Druckleitung durch Schließen des zweiten Sperrventils geschlossen. Auf diese Weise können auch Proben, die mit hohem Druck in der Druckleitung geprüft werden, mit einem einzigen Kolbenhub des hydraulischen Kolbens untersucht werden.

Die erfindungsgemäße Prüfanordnung, insbesondere, wenn sie eine Querschnittsverengung aufweist, ist sehr vorteilhaft, weil sie mit geringem Flüssigkeitseinsatz, meist 50 ml bis 100 ml, in der Druckleitung betrieben wird und weil sie mit einem Stromanschluss von üblichen 220 V Wechselstrom und einem Arbeitsstrom von 24 V Gleichstrom betrieben werden kann. Aufgrund dieser Voraussetzungen ist die erfindungsgemäße Prüfanordnung außerordentlich betriebssicher und kann überall betrieben werden, insbesondere z. B. in der Nähe von Produktionsanlagen zur Qualitätsüberwachung.

Nach einer Weiterbildung der Erfindung weist die Prüfanordnung eine Steuerung auf. Die Steuerung ist vorteilhaft so ausgelegt, dass sie den Antrieb und optional das Fluten der Druckleitung durch Betätigen des Einlauf- und des Auslaufventils steuert. Bevorzugt werden auch die weiteren Ventile der Prüfanordnung durch die Steuerung gesteuert. Auf diese Weise kann ein vollständiger Prüfzyklus durch die Steuerung automatisiert gesteuert werden. Dabei können die Ventile entweder nach einer Zeitsteuerung gesteuert werden oder das Öffnen und Schließen der Ventile wird nach Rückmeldung von Sensoren gesteuert, die z. B. das vollständige Fluten der Druckleitung melden.

Die Steuerung weist vorteilhaft einen Speicher auf, in dem verschiedene Prüfzyklen hinterlegt sind, z. B. einen Prüfzyklus für den Drucktransmitter, der zum Erfassen niedriger Drücke geeignet ist oder einen Prüfzyklus für den Drucktransmitter, der zum Erfassen höherer Drücke geeignet ist. In Abhängigkeit von der zu prüfenden Probe kann die Geschwindigkeit des Druckaufbaus verschieden gestaltet sein, auch bei Messung mit dem gleichen Drucktransmitter. Alternativ oder in Kombination zur Geschwindigkeit des Druckaufbaus kann der maximale Druck, bis zu dem der Druckaufbau erfolgt, individuell für eine spezifische Probe gewählt werden.

Besonders bevorzugt ist es, wenn die Steuerung durch ein Ein- und Ausgabe-Display ergänzt wird. Durch Eingaben am Display wird ein Prüfprofil gestartet und ein Ausgabe-Display kann das Ergebnis der Prüfung und/oder den Verlauf der Prüfung anzeigen, aber auch aufzeichnen und ggf. ausgeben, z. B. ausdrucken oder an einen Speicher übermitteln, so dass ein Protokoll der Prüfung erstellt wird.

Das erfindungsgemäße Verfahren zum Prüfen einer Probe mit einer Prüfungsanordnung so, wie sie vorstehend beschrieben ist, mit einem Prüfmittel und mit einer zu prüfenden Probe mit den Schritten:
- Öffnen des Einlauf- und des Auslaufventils
- Fluten der Druckleitung mit einem Fluid
- Schließen des Einlauf- und optional des Auslaufventils
- Starten des Antriebs
- Steuern des Antriebs nach einem vorgegebenen Profil, insbesondere nach einem Druckprofil, einem Kraftprofil oder einem Drehzahlprofil,
- Erfassen des Druckabfalls.

Das Verfahren zum Prüfen einer Probe mit der erfindungsgemäßen Prüfungsanordnung wird gestartet, indem das Einlauf- und das Auslaufventil geöffnet werden, so dass die Druckleitung geflutet werden kann. Das Fluid, in der Regel Wasser oder Öl, wird aus einem Reservoir, d. h., aus einem Behälter oder einer Versorgungsleitung in die Druckleitung gespeist. Das Reservoir kann eine übliche Wasserleitung sein, bevorzugt ist das Reservoir ein Behälter und das Fluid wird für mehrere Messungen im Kreislauf geführt.

Optional, insbesondere, wenn eine Kolben-Zylinder-Anordnung zum Druckaufbau in der Druckleitung installiert ist, wird ein stromab der Kolben-Zylinder-Anordnung angeordnetes Sperrventil geschlossen, so dass das Fluid nicht durch den Saugdruck der Kolben-Zylinder-Anordnung aus der Druckleitung in Richtung auf das Einlaufventil gefördert wird. Erst wenn ein Druckausgleich erfolgt ist und damit gewährleistet wird, dass das Fluid wie vorgesehen in Strömungsrichtung durch die Druckleitung fließt und diese füllt, wird das Sperrventil geöffnet.

Nach dem Fluten, d. h. dem vollständigen Füllen der Druckleitung mit dem Fluid, wird das Einlaufventil wieder geschlossen. Das Auslaufventil wird nur geschlossen, wenn der Antrieb kraftgesteuert ist. Bei einem drehzahlgesteuerten Antrieb ist die Druckleitung stromab der zu prüfenden Probe so weit verengt, dass das Auslaufventil geöffnet bleibt. Der Antrieb, kraft- oder drehzahlgesteuert, wird gestartet. Der Druck in der Druckleitung und in dem an das zweite Ende der Druckleitung angeschlossenen Prüfmittel mit der Probe steigt, entweder durch die vom Antrieb angetriebene Pumpe oder die vom Antrieb angetriebene Kolben-Zylinder-Anordnung. Der Antrieb wird nach einem vorgegebenen Profil gesteuert, insbesondere nach einem Druckprofil, einem Kraftprofil oder einem Drehzahlprofil. Der Antrieb steigert den Druck in der Druckleitung auf ein vorgegebenes Maximum, wobei die Geschwindigkeit der Drucksteigerung und das Maximum des Drucks individuell einstellbar sind. Der in der Druckleitung herrschende Druck wird von einem Drucktransmitter erfasst und an die Steuerung weitergegeben.

Optional wird der Verlauf der Prüfung angezeigt oder aufgezeichnet. Bei Versagen der Probe wird der Druckabfall, der sich durch das Zerbrechen der Probe einstellt, erfasst. Versagt die Probe nicht, weil sie einem vorgegebenen maximalen Druck standhält, wird erfasst, dass die Probe diesem Druck standhält.

Weist die Prüfanordnung zwei Drucktransmitter, z. B. für unterschiedliche Drücke, auf, so ist in Strömungsrichtung vor jedem Drucktransmitter ein Sperrventil angeordnet. Mit Beginn der Prüfung wird manuell oder durch die Steuerung ein Sperrventil geöffnet, so dass der für die Prüfung benötigte Drucktransmitter in die Druckleitung eingebunden ist. Wird ein Drucktransmitter, der zum Messen niedriger Drücke ausgelegt ist, nicht benötigt, so bleibt das stromaufwärts vom Drucktransmitter angeordnete Sperrventil geschlossen. Zusätzlich wird ein stromabwärts vom Drucktransmitter angeordnetes, weiteres Sperrventil geschlossen, damit gewährleistet ist, dass dieser Drucktransmitter nicht durch einen zu hohen Druck in der Druckleitung beschädigt wird.

Wird eine Probe mit hohem Druck geprüft, so kann optional die Prüfanordnung verbessert werden, indem eine Strömungsverengung stromabwärts des Anschlusses für das Prüfmittel und die Probe in einer Verzweigung der Druckleitung angeordnet wird. Ein Sperrventil schließt die Druckleitung, so dass das Fluid durch die Strömungsverengung strömen muss. Dadurch baut sich unmittelbar ein weitaus höherer Druck in der Druckleitung auf als ohne die Strömungsverengung. Diese Maßnahme ermöglicht es, auch mit einem Hydraulikzylinder mit kleinerem Hub die Messung einer Probe bei höherem Druck durchzuführen.

An die Prüfanordnung können verschiedene Prüfmittel angeschlossen werden. Ein besonders geeignetes Prüfmittel, das an die Prüfanordnung angeschlossen werden kann, wird nachfolgend beschrieben. Dieses Prüfmittel weist einen zylindrischen Grundkörper aus Metall auf, der mit einer zentrischen Bohrung versehen ist. Der Grundkörper wird auch als Hülse bezeichnet. Von der zentrischen Bohrung aus erstrecken sich radiale Auslassöffnungen bis zur Oberfläche des Grundkörpers. Der Grundkörper weist ein erstes geschlossenes Ende und ein zweites mit einer Zuleitung für ein Fluid versehenes Ende auf. Das zweite Ende ist vorteilhaft mit einem ersten Kupplungsteil versehen, das mit einem zweiten, korrespondierenden Kupplungsteil an der Prüfanordnung korrespondiert. Eine Dichtung ist über den Grundkörper gespannt, mindestens über dem Abschnitt des Grundkörpers, der Auslassöffnungen aufweist. Die Dichtung ist typisch aus einem flüssigkeitsdichten Material. Es kann sich um eine Einmaldichtung handeln, es kann aber auch eine Dichtung sein, die wiederholt verwendet wird.

Die Dichtung wird vorteilhaft über einen Vorsprung oder eine Hinterschneidung bzw. Nut am Grundkörper gezogen und dort durch Verpressen mit einer Mutter gesichert, die mit einem Gewinde auf der Außenfläche des Grundkörpers im Eingriff steht. Am ersten Ende des Grundkörpers ist die Mutter als geschlossene Kappe ausgebildet, am zweiten Ende des Grundkörpers umschließt die Mutter die Außenfläche des Grundkörpers. Bevorzugt ist in die Hinterschneidung oder Nut oder auf den Vorsprung ein O-Ring aus elastischem Material aufgesetzt, an dem die Mutter nach dem Festsetzen der Dichtung anliegt.

Auf diese Weise hat das Prüfmittel eine glatte zylindrische Oberfläche und die Dichtung kann ohne großen mechanischen Aufwand hergerichtet werden. Damit ist eine wesentliche Fehlerquelle beim Herrichten des Prüfmittels ausgeschlossen.

Eine Probe des zu prüfenden Materials ist zylindrisch gestaltet und weist einen Innendurchmesser auf, der geringfügig größer ist als der Außendurchmesser des Grundkörpers bzw. der Muttern, die die Dichtung fixieren. Die Probe kann aus einer dünnen Schicht bestehen, die Bestandteil eines komplexen keramischen Produkts werden soll oder es kann sich um eine Probe größerer Dicke handeln, die in der später herzustellenden Materialstärke geprüft wird. Die Probe wird nach einer vorteilhaften Ausführung unmittelbar auf dem Prüfmittel ausgebildet, z. B. durch Eintauchen des Prüfmittels in eine Suspension. Die sich auf dem Prüfmittel ablagernde Schicht wird getrocknet und als Probe geprüft.

Das Prüfmittel mit der darauf aufgeschobenen Probe oder mit der direkt auf dem Prüfmittel ausgebildeten Probe wird an die Prüfanordnung angekoppelt, so dass die Bohrung und die Auslassöffnungen mit der Druckleitung in Verbindung stehen. Prüfanordnung bzw. Druckleitung und Prüfmittel werden mit Fluid gefüllt und anschließend wird der Druck des Fluids auf ein Maximum erhöht. Hält die Probe dem Druck nicht stand, so erfasst vorteilhaft die Steuerung den Druck, bei dem die Probe versagt. Aus diesem Druck kann die Druckspannung errechnet werden, die das Material der Probe aushält. Der Vorteil des Prüfmittels liegt insbesondere darin, dass die Innenfläche der Probe insgesamt gleichmäßig mit Druck beaufschlagt wird.

Details der Erfindung werden an Hand der nachfolgenden Ausführungsbeispiele näher erläutert. Es zeigen:
- Fig. 1: eine Ansicht des an der Rückseite geöffneten Gehäuses der erfindungsgemäßen Prüfanordnung in schematischer Darstellung,
- Fig. 2: ein seitlicher Schnitt durch die erfindungsgemäße Prüfanordnung in schematischer Darstellung,
- Fig. 3: eine Vorderansicht der erfindungsgemäßen Prüfanordnung in schematischer Darstellung ohne Abdeckplatte,
- Fig. 4: eine schematische Darstellung der Druckleitung mit den darin angeordneten Komponenten,
- Fig. 5: eine Ansicht der Vorderseite des Gehäuses
- Fig. 6: ein Schnitt durch ein Prüfmittel für die erfindungsgemäße Prüfanordnung sowie
- Fig. 7: weitere Bestandteile des Prüfmittels nach Fig. 5
- Fig. 8: eine Ansicht eines Prüfmittels mit Zubehör

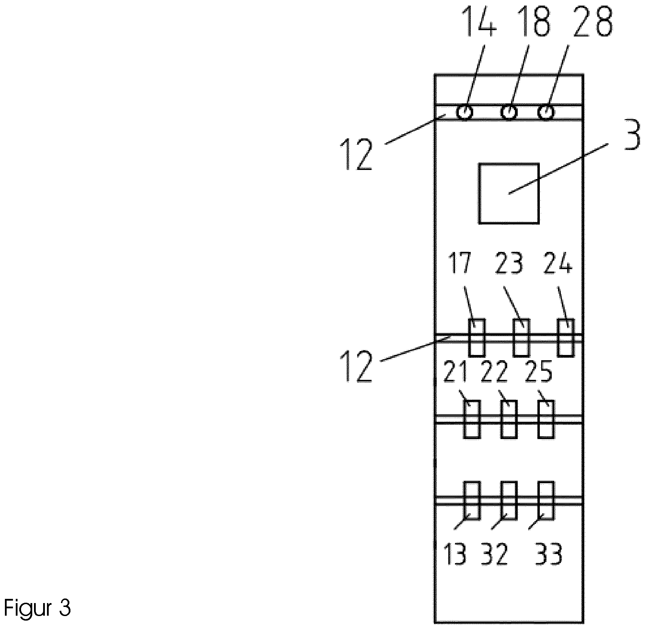

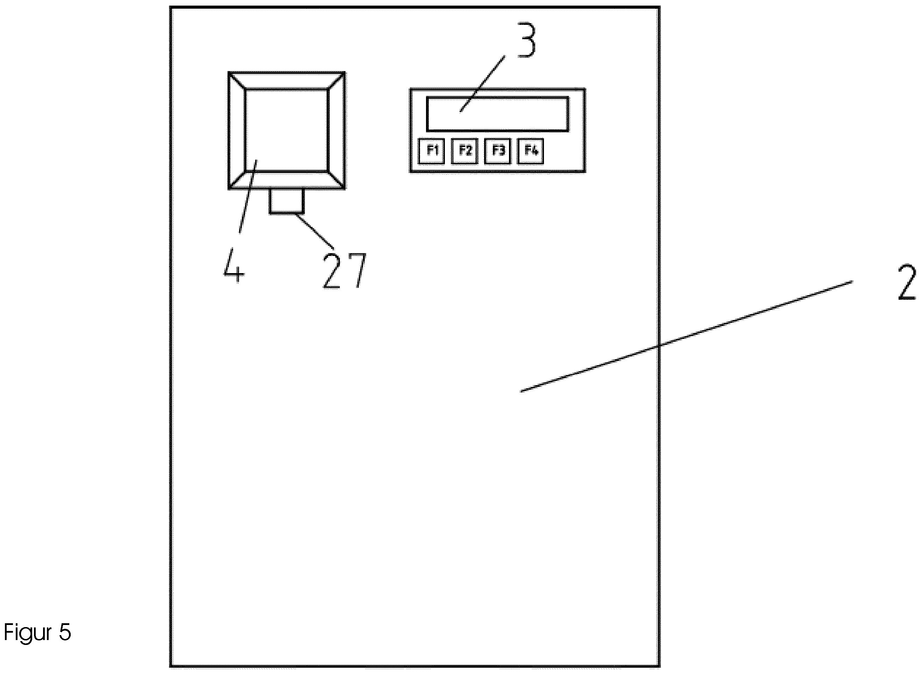

Die Prüfanordnung ist als Gehäuse 1 ausgeführt, auf dessen Vorderseite 2 gemäß Fig. 3 und Fig. 5 ein Bedienfeld 3 und optional ein Anzeigefeld sowie ein Anschluss 4 für ein Prüfmittel angebracht sind. Der Anschluss 4 ist vorteilhaft als Hydraulikschnellkupplung ausgeführt.

Das Bedienfeld 3 weist hier vier Tasten auf, wobei Taste F1 das Beladen der Prüfungsanordnung bzw. der Druckleitung mit einem Fluid auslöst. Als Fluid wird hier Wasser eingesetzt, alternativ kann aber auch ein Öl eingesetzt werden. Taste F₂ startet ein Prüfprofil mit einem maximalen Druck von 2 bar. Taste F3 startet ein Prüfprofil mit einem maximalen Druck von 16 bar. Taste F4 bewirkt, dass die Prüfanordnung drucklos gestellt wird. Sämtliche Ventile sind geöffnet. Diese Stellung dient entweder zum Anschließen eines Prüfmittels oder zum Abbrechen einer Prüfung.

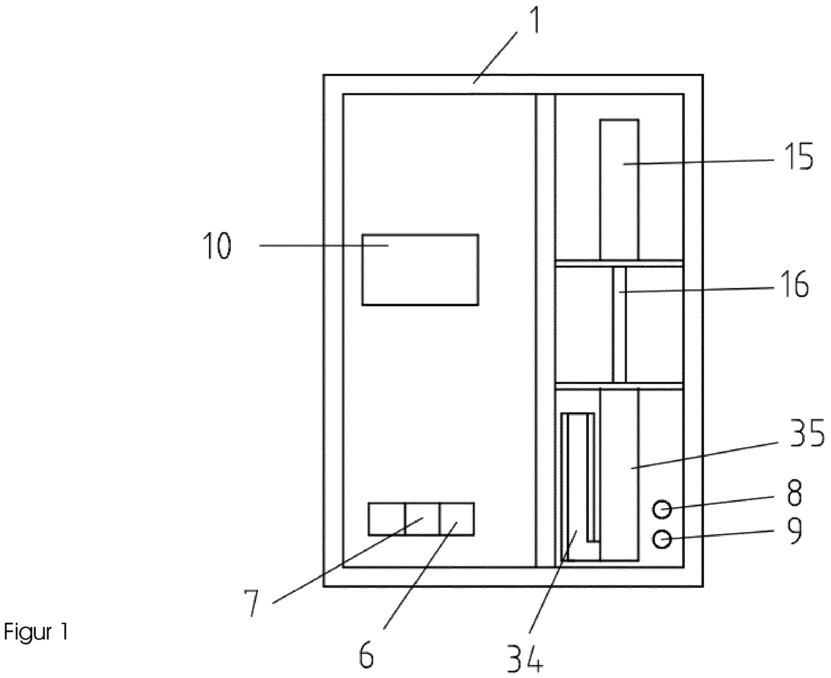

Die Sicht in das an der Rückseite 5 geöffneten Gehäuses 1 der Prüfanordnung zeigt gemäß Fig. 1 einen Anschluss 6 für die Energieversorgung und einen Anschluss 7 für eine Datenleitung. Weiter zeigt die Rückseite 5 einen Einlauf 8 und einen Auslauf 9 für Wasser. Einlauf 8 und Auslauf 9 sind an einen Behälter 11 angeschlossen, so dass das Wasser für mehrere Messungen nacheinander im Kreislauf geführt wird. Fig. 1 zeigt weiter den drehzahlgesteuerten Antrieb 34 mit einer Leistung von 110 W, der an einen 220 V Wechselstromanschluss angeschlossen und mit 24 V Gleichstrom betrieben wird, und der mit der elektrischen Kolben-Zylinder-Anordnung 35 in Verbindung steht sowie die hydraulische Kolben-Zylinder-Anordnung 15, deren Kolben 16 mit dem Kolben der elektrischen Kolben-Zylinder-Anordnung 35 fest verbunden ist.

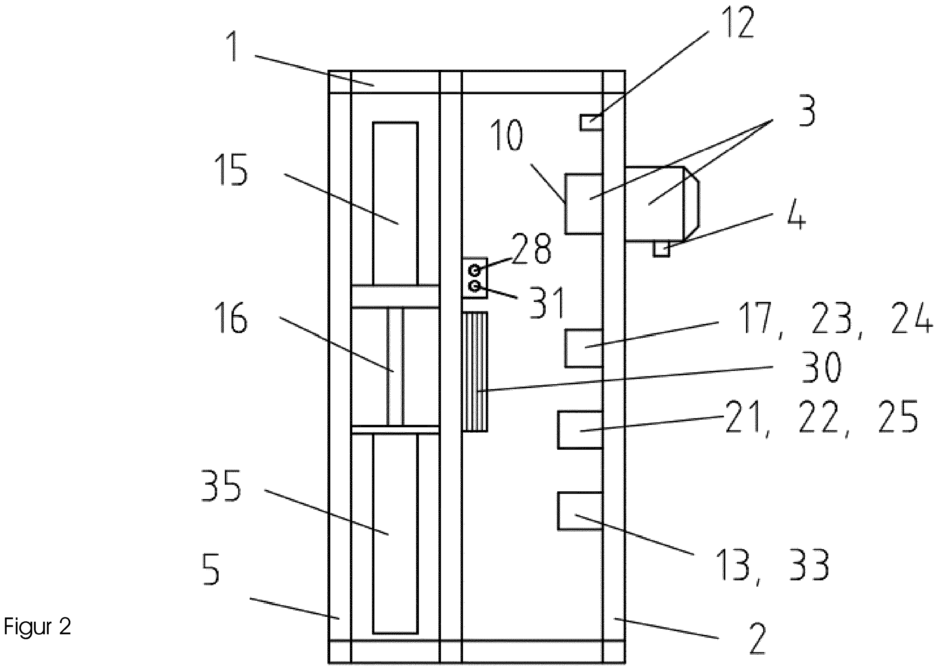

Der seitliche Schnitt durch die Prüfanordnung gemäß Fig. 2 zeigt eine Steuerung 10, die mit dem Bedienfeld 3 in Verbindung steht. Mittels der Tasten F1 bis F4 des Bedienfelds 3 wird die Steuerung 10 aktiviert (vgl. Fig. 3). Optional sendet die Steuerung 10 Informationen über den Ablauf der Prüfung an ein Anzeigefeld.

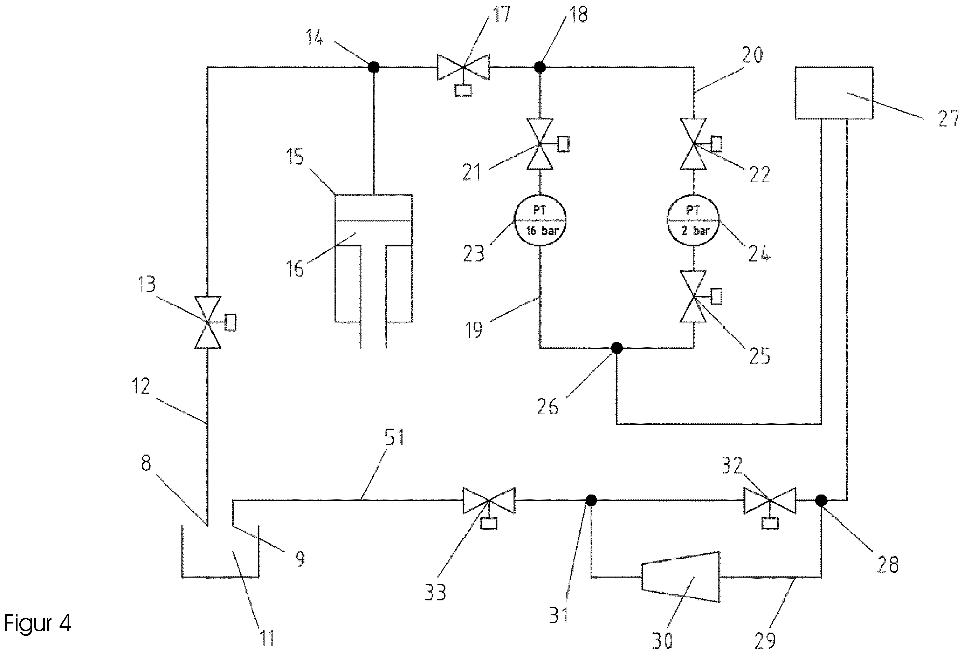

Fig. 3 zeigt das Bedienfeld 3 mit den Bedientasten F1 bis F4. Fig. 3 zeigt weiter die Druckleitung 12 mit der ersten Verzweigung 14, der zweiten Verzweigung 18 und der vierten Verzweigung 28, die in die Druckleitung 12 eingesetzt sind. Weiter zeigt Fig. 2 die Drucktransmitter 23 und 24, die ebenfalls in die Druckleitung 12 eingesetzt sind. In die Druckleitung 12 sind zudem das Einlaufventil 13 und das Auslaufventil 33 eingesetzt. Das Zusammenwirken dieser Komponenten wird in Fig. 4 näher erläutert.

Fig. 4 zeigt in schematischer Darstellung den Behälter 11, der in die Druckleitung 12 eingesetzt ist. Die Druckleitung 12 ist aus Edelstahl oder aus Kunststoff, z. B. aus Polyamid, und weist einen Innendurchmesser von 4 mm auf. Die Druckleitung 12 mündet am fünften Sperrventil 32 oder am Auslaufventil 33 in die drucklose Auslaufleitung 51. Der Einlauf 8 in die Druckleitung 12 und der Auslauf 9 aus der drucklosen Auslaufleitung 51 setzten am Behälter 11 an, so dass ein geschlossener Kreislauf gewährleistet ist. Die Druckleitung 12 weist ein Volumen von 80 ml auf. Die durch Pfeile angedeutete Strömungsrichtung erstreckt sich vom Einlauf 8 zum Auslauf 9. Stromab des Einlaufs 8 ist das Einlaufventil 13 in die Druckleitung 12 eingesetzt. Es ist -wie auch die im Folgenden beschriebenen Ventile- ein Magnetventil, das mit der Steuerung 10 in Verbindung steht und von dieser geöffnet und geschlossen wird.

Nach dem Einlaufventil 13 ist eine Kolben-Zylinder-Anordnung 15, 35 mittels einer ersten Verzweigung 14 in die Druckleitung 12 eingesetzt, von der in Fig. 4 die hydraulische Kolben-Zylinder-Anordnung 15 dargestellt ist. Der Kolben 16 der hydraulischen Kolben-Zylinder-Anordnung 15 ist mit dem hier nicht dargestellten Kolben der elektrischen Kolben-Zylinder-Anordnung fest verbunden. Die elektrische Kolben-Zylinder-Anordnung ist mit dem hier nicht dargestellten drehzahlgesteuerten Antrieb verbunden. Der Antrieb 34 und die elektrische Kolben-Zylinder-Anordnung 35 werden in Fig. 1 gezeigt. Alternativ kann die Kolben-Zylinder-Anordnung 15, 35 durch eine Pumpe ersetzt werden.

Stromab der hydraulischen Kolben-Zylinder-Anordnung 15 kann optional ein erstes Sperrventil 17 in die Druckleitung 12 eingesetzt sein, dass beim Füllen der Druckleitung 12 zunächst geschlossen ist, um den Saugdruck zu kompensieren, den der Hub der hydraulischen Kolben-Zylinder-Anordnung 15 auf die Druckleitung 12 ausübt. Das erste Sperrventil 17 ist lediglich zu Beginn des Füllvorgangs der Druckleitung 12 geschlossen; in allen anderen Betriebszuständen der Prüfanordnung ist das erste Sperrventil 17 geöffnet. Alternativ kann die Funktion des ersten Sperrventils 17 durch das zweite und das dritte Sperrventil 21, 22 übernommen werden, das erste Sperrventil 17 entfällt dann.

Stromabwärts des ersten Sperrventils 17 ist eine zweite Verzweigung 18 in die Druckleitung 12 eingebaut, so dass die Druckleitung 12 in die erste Zweigleitung 19 und in die zweite Zweigleitung 20 aufgeteilt ist. Die Zweigleitungen 19 und 20 verlaufen parallel. In jede Zweigleitung 19, 20 ist ein Sperrventil eingesetzt, das zweite Sperrventil 21 in die Zweigleitung 19 und das dritte Sperrventil 22 in die Zweigleitung 20. Stromabwärts der Sperrventile 21, 22 ist jeweils ein Drucktransmitter in die Zweigleitungen 19, 20 eingesetzt. Ein erster Drucktransmitter 23 ist in die Zweigleitung 19 eingesetzt. Er erfasst einen Druck bis 16 bar. Ein zweiter Drucktransmitter 24 ist in die Zweigleitung 20 eingesetzt. Er erfasst einen Druck bis 2 bar. Steigt der Druck in der Druckleitung 12 - und damit auch in den Zweigleitungen 19, 20, so würde der Drucktransmitter 24 bei einem Druck von mehr als 2 bar beschädigt werden. Um dies zu verhindern, ist ein viertes Sperrventil 25 stromabwärts des zweiten Drucktransmitters 24 in die zweite Zweigleitung 20 eingesetzt.

Wird eine Probe mit einem Druck von mehr als 2 bar gemessen, so sind das dritte und das vierte Sperrventil 22, 25 geschlossen, um den Drucktransmitter 24 zu entlasten und das zweite Sperrventil 21 ist geöffnet. Der während des Prüfvorgangs in der Druckleitung 12 und der Zweigleitung 19 herrschende Druck wird nunmehr vom Drucktransmitter 23 erfasst und an die Steuerung 10 übermittelt. Wird eine Probe mit einem Druck von weniger als 2 bar gemessen, bleibt das zweite Sperrventil 21 geschlossen und das dritte und das vierte Sperrventil 22, 25 sind geöffnet. Der Drucktransmitter 24 erfasst jetzt den Druck von maximal 2 bar, der in der Druckleitung 12 und der zweiten Zweigleitung 20 herrscht und übermittelt die gemessenen Werte an die Steuerung 10.

Stromabwärts des Drucktransmitters 23 und des vierten Sperrventils 25 werden die erste und die zweite Zweigleitung 19, 20 an einer dritten Verzweigung 26 wieder zu einer einheitlichen Druckleitung 12 zusammengeführt. In diese Druckleitung 12 ist stromabwärts der Drucktransmitter 23, 24 eine hydraulische Schnellkupplung 27 als Auslass 4 für das Anschließen eines hier nicht dargestellten Prüfmittels eingesetzt. Es können verschiedene Prüfmittel an die Prüfanordnung angeschlossen werden; ein besonders geeignetes Prüfmittel ist nachfolgend näher erläutert.

Stromabwärts der hydraulischen Schnellkupplung 27 ist eine vierte Verzweigung 28 in die Druckleitung 12 eingefügt, so dass eine dritte Zweigleitung 29 parallel zur Druckleitung 12 verläuft. In die dritte Zweigleitung 29 ist eine Querschnittsbegrenzung, hier eine Kapillare 30 eingesetzt. An einer fünften Verzweigung 31 endet die dritte Zweigleitung 29 wieder in einer drucklosen Auslaufleitung 51. Zwischen der vierten Verzweigung 28 und der stromabwärts eingesetzten fünften Verzweigung 31 ist ein fünftes Sperrventil 32 in die Druckleitung 12 eingesetzt, um diese während eines Prüfvorgangs gegenüber der Auslaufleitung 51 abzuschließen. Von der fünften Verzweigung 31 erstreckt sich die drucklose Auslaufleitung 51 zu einem optional in die Auslaufleitung 51 einsetzbaren Auslaufventil 33, das beim Füllen und Leeren der Druckleitung 12 geöffnet ist, das aber während eines Prüfvorgangs geschlossen ist. Vom Auslaufventil 33 erstreckt sich die drucklose Auslaufleitung 51 zum Auslauf 9. Alternativ erstreckt sich die drucklose Auslaufleitung 51 vom fünften Sperrventil 32 zum Auslauf 9. Das Auslaufventil 33 ist nur dann erforderlich, wenn eine Prüfanordnung ohne Querschnittsverengung gebaut wird.

Die Querschnittsbegrenzung wird benötigt, wenn eine Probe mit hohem Druck gemessen wird. Die Prüfung, also das Erhöhen des Druckes in der Druckleitung 12, soll mit einem Hub des hydraulischen Kolbens 16 erfolgen. Bei niedrigem Druck ist diese unproblematisch. Um den Einsatz einer unnötig großen Kolben-Zylinder-Anordnung zu vermeiden, wird bei der erfindungsgemäßen Prüfungsanordnung beim Prüfen mit hohem Druck das vierte Sperrventil 25 geschlossen. In diesem Moment bewirkt die auf den Durchmesser der Kapillare 30 verengte Druckleitung 12 einen überproportionalen Druckanstieg in der Druckleitung 12 stromauf der Kapillare 30. Auf diese Weise kann auch mit einer kleineren Kolben-Zylinder-Anordnung eine Prüfung mit hohem Druck mit nur einem Kolbenhub durchgeführt werden.

Sämtliche Ventile der vorstehend beschriebenen Prüfanordnung, die beiden Drucktransmitter 23, 24 und die Kolben-Zylinder-Anordnung mit dem Antrieb sind mit der Steuerung 10 verbunden und werden von der Steuerung 10 gesteuert. Vorteilhaft sind in einem Speicher der Steuerung 10 verschiedene Prüfprofile, z. B. für hohen Druck und für niedrigen Druck hinterlegt, die z. B. durch die Tasten F1-F4 des Bedienfeldes 3 abgerufen werden können.

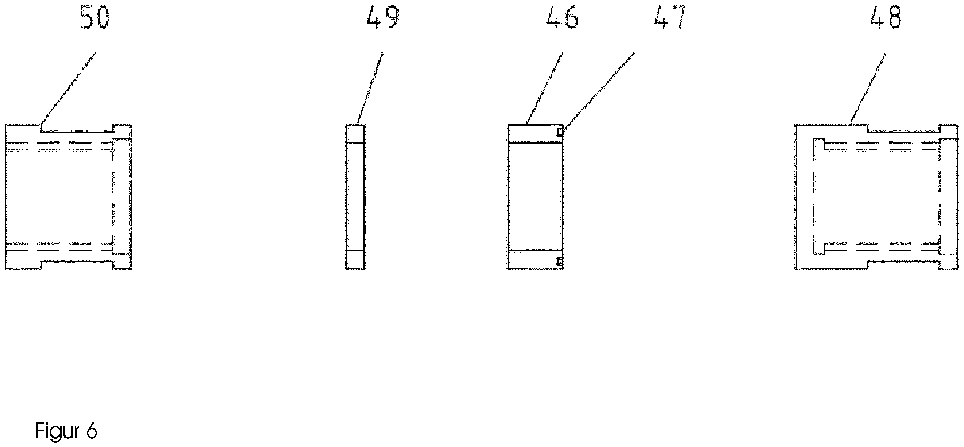

An die Prüfanordnung können verschiedene Prüfmittel angeschlossen werden. Ein besonders geeignetes Prüfmittel 36, das an die Prüfanordnung angeschlossen werden kann, ist in Fig. 6 abgebildet und wird nachfolgend beschrieben. Dieses Prüfmittel 36 weist einen zylindrischen Grundkörper 37 aus Metall auf, der mit einer zentrischen Sackbohrung 38 parallel zur Längsachse des Prüfmittels 36 versehen ist. Der Grundkörper 37 wird auch als Hülse bezeichnet. Von der zentrischen Bohrung 38 aus erstrecken sich radiale Auslassöffnungen 39 bis zur Oberfläche 40 des Grundkörpers 37. Der Grundkörper 37 weist ein erstes geschlossenes Ende 41 und ein zweites mit einer Zuleitung für ein Fluid versehenes Ende 42 auf. Das zweite Ende 42 ist vorteilhaft mit einem ersten Kupplungsteil versehen, das mit einem zweiten, korrespondierenden Kupplungsteil 27 (vgl. Fig. 4, 5) an der Prüfanordnung korrespondiert.

Eine Dichtung 43 ist über den Grundkörper 37 gespannt, mindestens über dem Prüfabschnitt 44 des Grundkörpers 37, der Auslassöffnungen aufweist. Die Dichtung 43 ist typisch aus einem flüssigkeitsdichten Material. Es kann sich um eine Einmaldichtung handeln, es kann aber auch eine Dichtung sein, die wiederholt verwendet wird. Die Dichtung 43 wird vorteilhaft über einen Vorsprung oder eine Hinterschneidung 45 bzw. Nut am Grundkörper 37 gezogen und dort gesichert.

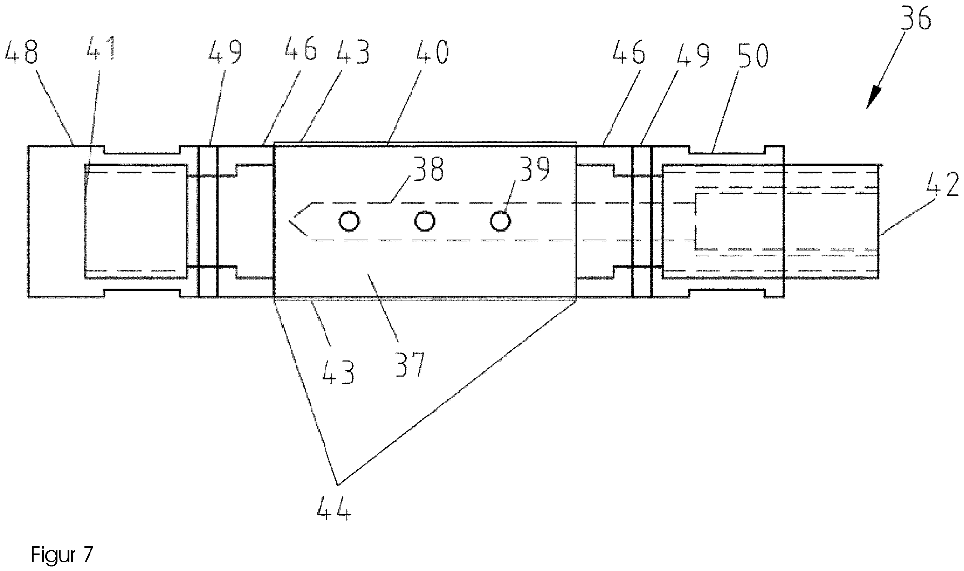

Das Sichern der Dichtung 43 erfolgt am ersten Ende 41 des Grundkörpers 37 durch Verpressen mit einer Hülse 46, die mit einer kreisringförmigen Ausnehmung 47 für einen O-Ring versehen ist (vgl. Fig. 7). Der O-Ring liegt beim einsatzbereit montierten Prüfmittel 36 an der Dichtung 43 an. Die Hülse 46 wird durch eine Gewindemutter 48 gesichert, die mit einem Gewinde auf der Oberfläche 40 des Grundkörpers 37 im Eingriff steht. Die Gewindemutter 48 sowie die weiteren Bestandteile des Prüfmittels 36 sind in Fig. 6 dargestellt. Die Gewindemutter 48 ist als geschlossene Kappe ausgebildet, die die Hülse 46 gegen die Hinterschneidung 45 am Grundkörper 37 drückt, um die Dichtung 43 zu sichern.

Am zweiten Ende 42 des Grundkörpers 37 wird die Dichtung 43 durch einen Gleitring 49 gesichert, der seinerseits durch eine beidseitig offene Gewindemutter 50 am Grundkörper 37 gesichert wird.

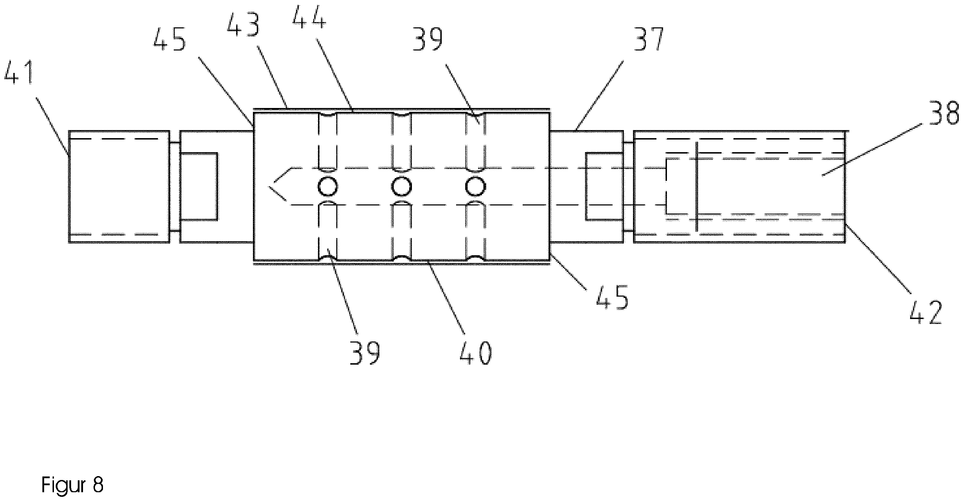

Auf diese Weise hat das Prüfmittel 36, wie in Fig. 8 dargestellt, eine glatte zylindrische Oberfläche 40 und die Dichtung 43 kann ohne großen mechanischen Aufwand hergerichtet werden. Damit ist eine wesentliche Fehlerquelle beim Herrichten des Prüfmittels 36 ausgeschlossen.

Eine Probe des zu prüfenden Materials ist zylindrisch gestaltet und weist einen Innendurchmesser auf, der geringfügig größer ist als der Außendurchmesser des Grundkörpers 37 bzw. der Gewindemuttern 48, 50, die die Dichtung 43 fixieren. Sie wird bevorzugt hergestellt durch das Eintauchen des Prüfmittels 36 in eine Suspension, die sich ggf. nach Trocknung als Probe auf der Oberfläche 40 des Prüfmittels 36 ablagert.

Das Prüfmittel 36 mit der darauf aufgeschobenen oder abgelagerten Probe wird an die Prüfanordnung angekoppelt, so dass die Bohrungen 38, 39 mit der Druckleitung 12 in Verbindung stehen. Prüfanordnung bzw. Druckleitung 12 und Prüfmittel 36 werden mit Fluid gefüllt und anschließend wird der Druck des Fluids auf ein Maximum erhöht. Hält die Probe dem Druck nicht stand, so erfasst vorteilhaft die Steuerung 10 durch Signale der Drucktransmitter 23 oder 24 den Druck, bei dem die Probe versagt. Aus diesem Druck kann die Druckspannung errechnet werden, die das Material der Probe aushält. Der Vorteil des Prüfmittels 36 liegt insbesondere darin, dass die Innenfläche der Probe insgesamt gleichmäßig mit Druck beaufschlagt wird und dass das Montieren des Prüfmittels einfach und fehlerfrei erfolgen kann.

**Bezugszeichenliste**

| | | | |
|---|---|---|---|
| 1 | Gehäuse | 30 | Kapillare |
| 2 | Vorderseite | 31 | Fünfte Verzweigung |
| 3 | Bedienfeld | 32 | Fünftes Sperrventil |
| 4 | Anschluss | 33 | Auslaufventil |
| 5 | Rückseite | 34 | drehzahlgetriebener Antrieb |
| | | | |
| 6 | Anschluss für die Energieversorgung | 35 | elektrische Kolben-Zylinder-Anordnung |
| 7 | Anschluss für eine Datenleitung | | |
| 8 | Einlauf für Wasser | 36 | Prüfmittel |
| 9 | Auslauf für Wasser | 37 | Grundkörper |
| 10 | Steuerung | 38 | zentrische Sackbohrung |
| 11 | Behälter | 39 | radiale Bohrung |
| 12 | Druckleitung | 40 | Oberfläche |
| 13 | Einlaufventil | 41 | erstes Ende |
| 14 | Erste Verzweigung | 42 | zweites Ende |
| 15 | Hydraulische Kolben-Zylinder Anordnung | 43 | Dichtung |
| | | 44 | Prüfabschnitt |
| 16 | Kolben der hydraulischen | 45 | Hinterschneidung |
| | Kolben-Zylinder-Anordnung | 46 | Hülse |
| 17 | Erstes Sperrventil | 47 | Ausnehmung |
| 18 | Zweite Verzweigung | 48 | Gewindemutter |
| 19 | Erste. Zweigleitung | 49 | Gleitring |
| 20 | Zweite Zweigleitung | 50 | offene Gewindemutter |
| 21 | Zweites Sperrventil | 51 | drucklose Auslaufleitung |
| 22 | Drittes Sperrventil | F1-F4 | Bedientasten |
| 23 | Drucktransmitter 16 bar | | |
| 24 | Drucktransmitter 2 bar | | |
| 25 | Viertes Sperrventil | | |
| 26 | Dritte Verzweigung | | |
| 27 | Hydraulische Schnellkupplung | | |
| 28 | Vierte Verzweigung | | |
| 29 | Dritte Zweigleitung | | |

## Patentansprüche

1. Prüfanordnung zum Erfassen der Bruchspannung, wobei die Prüfanordnung ein Prüfmittel (36) für eine Probe und einen Antrieb (34), der drehzahlgesteuert ist umfasst und der nach einer ersten Alternative mit einer Kolben-Zylinder-Anordnung (15, 35) und nach einer zweiten Alternative mit einer Pumpe verbunden ist, wobei die Kolben-Zylinder-Anordnung (15, 35) oder die Pumpe an einer Druckleitung (12) angeschlossen ist, wobei die Druckleitung (12) einen Anschluss (4) für das Prüfmittel (36) aufweist und wobei die Kolben-Zylinder-Anordnung (15, 35) eine mit dem Antrieb (34) verbundene elektrische Kolben-Zylinder-Anordnung (35) und eine zwischen der elektrischen Kolben-Zylinder-Anordnung (35) und der Druckleitung (12) angeordnete hydraulische Kolben-Zylinder-Anordnung (15) aufweist, wobei der Elektrozylinder und der Hydraulikzylinder über fest miteinander verbundene Kolben (16) verbunden sind,
**dadurch gekennzeichnet, dass** eine Querschnittsbegrenzung (30) vorgesehen ist, die in einer Verzweigung (28) der Druckleitung parallel zur Druckleitung angeordnet ist, wobei die Verzweigung in Strömungsrichtung nach der zu prüfenden Probe angebracht ist, und die Druckleitung stromab der Verzweigung mit einem Sperrventil (32) abgeschlossen werden kann.

2. Prüfanordnung nach Anspruch 1, **dadurch gekennzeichnet, dass** ein Einlaufventil (13) und ein Auslaufventil (33) in die Druckleitung (12) eingesetzt sind.

3. Prüfanordnung nach mindestens einem der Ansprüche 1 oder 2, **dadurch gekennzeichnet, dass** mindestens ein Drucktransmitter (23, 24) in die Druckleitung (12) eingesetzt ist, der den in der Druckleitung (12) herrschenden Druck erfasst.

4. Prüfanordnung nach Anspruch 1 bis 3, **dadurch gekennzeichnet, dass** mindestens zwei Drucktransmitter (23, 24), die verschiedene Drücke erfassen, in die Druckleitung (12) eingesetzt sind, denen jeweils stromauf ein Sperrventil (21, 22) vorgeschaltet ist.

5. Prüfanordnung nach Anspruch 4, **dadurch gekennzeichnet, dass** stromabwärts des Drucktransmitters (24), der den niedrigeren Druck erfasst, ein Sperrventil (25) angeordnet ist.

6. Prüfanordnung nach mindestens einem der vorstehenden Ansprüche,
**dadurch gekennzeichnet, dass** die Prüfanordnung eine Steuerung (10) aufweist.

7. Prüfanordnung nach mindestens einem der Ansprüche 1 bis 6, **dadurch gekennzeichnet, dass** die Prüfanordnung ein Display (3) aufweist.

8. Prüfanordnung nach einem der Ansprüche 1 bis 7, mit einem Prüfmittel, aufweisend einen zylindrischen Grundkörper (37) mit einer zentrischen Sackbohrung (38) und mit einem Prüfabschnitt (44) mit radialen, bis zur Oberfläche des Grundkörpers reichenden Bohrungen (39) und mit einer Dichtung (43), die über den Prüfabschnitt (44) gespannt ist, **dadurch gekennzeichnet, dass** die Dichtung (43) durch eine Hülse (46) mit einem O-Ring sowie durch einen Gleitring (49) gesichert wird, die jeweils durch eine Gewindemutter (48, 50) gesichert werden.

9. Verfahren zum Prüfen einer Probe mit einer Prüfungsanordnung, nach einem der Ansprüche 1 bis 8 mit einer zu prüfenden Probe mit den Schritten:
- Öffnen des Einlauf- und des Auslaufventils (13, 33)
- Fluten der Druckleitung (12) mit einem Fluid
- Schließen des Einlauf und des Auslaufventils (13, 33)
- Starten des Antriebs (34)
- Steuern des Antriebs (34) nach einem vorgegebenen Profil, insbesondere nach einem Druckprofil, einem Kraftprofil oder einem Drehzahlprofil,
- Erfassen des Druckabfalls.

10. Verfahren nach Anspruch 9, **dadurch gekennzeichnet, dass** der Druckverlauf während des Prüfens aufgezeichnet wird.

11. Verfahren nach Anspruch 9 oder 10, **dadurch gekennzeichnet, dass** der Druckverlauf während des Prüfens angezeigt wird.

## Claims

1. A test assembly for capturing the fracture stress, wherein the test assembly comprises a test means (36) for a specimen and a drive (34), which is speed-controlled and which, according to a first alternative, is connected to a piston-cylinder assembly (15, 35) and, according to a second alternative, is connected to a pump, wherein the piston-cylinder assembly (15, 35) or the pump is joined to a pressure line (12), wherein the pressure line (12) has a connection (4) for the test means (36) and wherein the piston-cylinder assembly (15, 35) has an electric piston-cylinder assembly (35) connected to the drive (34) and a hydraulic piston-cylinder assembly (15) arranged between the electric piston-cylinder assembly (35) and the pressure line (12), wherein the electric cylinder and the hydraulic cylinder are connected via pistons (16) fixedly connected to one another,
**characterized in that** a cross-sectional delimitation (30) is provided, which is arranged in a branch (28) of the pressure line parallel to the pressure line, wherein the branch is mounted in the flow direction after the specimen to be tested, and the pressure line can be terminated with a shut-off valve (32) downstream of the branch.

2. The test assembly according to Claim 1, **characterized in that** an inlet valve (13) and an outlet valve (33) are inserted into the pressure line (12).

3. The test assembly according to at least one of Claims 1 or 2, **characterized in that** at least one pressure transmitter (23, 24) is inserted into the pressure line (12), which detects the prevailing pressure in the pressure line (12).

4. The test assembly according to Claim 1 to 3, **characterized in that** at least two pressure transmitters (23, 24), which detect various pressures, are inserted into the pressure line (12), upstream of each of which there is a shut-off valve (21, 22).

5. The test assembly according to Claim 4, **characterized in that** a shut-off valve (25) is arranged downstream of the pressure transmitter (24) which detects the lower pressure.

6. The test assembly according to at least one of the preceding claims, **characterized in that** the test assembly comprises a control system (10).

7. The test assembly according to at least one of Claims 1 to 6, **characterized in that** the test assembly comprises a display (3).

8. The test assembly according to one of Claims 1 to 7 with a test means, having a cylindrical base body (37) with a centrical blind borehole (38) and with a test section (44) having radial bores (39) extending up to the surface of the base body and with a seal (43) which is stretched over the test section (44), **characterized in that** the seal (43) is secured by a sleeve (46) having an O-ring as well as by a slide ring (49), each of which are secured by a threaded nut (48, 50).

9. A method for testing a specimen with a testing assembly, according to one of Claims 1 to 8, with a specimen to be tested, having the steps of:
- opening the inlet and outlet valve (13, 33),
- flooding the pressure line (12) with a fluid,
- closing the inlet and the outlet valve (13, 33),
- starting the drive (34),
- controlling the drive (34) according to a predefined profile, in particular according to a pressure profile, a force profile or a speed profile,
- detecting the pressure drop.

10. The method according to Claim 9, **characterized in that** the pressure curve is recorded during testing.

11. The method according to Claim 9 or 10, **characterized in that** the pressure curve is displayed during testing.

## Revendications

1. Dispositif d'essai destiné à détecter la tension de fracture, dans lequel le dispositif d'essai comporte un moyen d'essai (36) pour un échantillon et un entraînement (34), dont la vitesse de rotation est commandée et lequel est relié à un ensemble de piston-cylindre (15, 35) selon une première alternative et à une pompe selon une deuxième alternative, dans lequel l'ensemble de piston-cylindre (15, 35) ou la pompe est raccordé(e) à une conduite de pression (12), dans lequel la conduite de pression (12) présente un raccord (4) pour le moyen d'essai (36) et dans lequel l'ensemble de piston-cylindre (15, 35) présente un ensemble de piston-cylindre électrique (35) relié à l'entraînement (34) ainsi qu'un ensemble de piston-cylindre hydraulique (15) disposé entre l'ensemble de piston-cylindre électrique (35) et la conduite de pression (12), dans lequel le cylindre électrique et le cylindre hydraulique sont reliés par des pistons (16) reliés fixement entre eux,
**caractérisé en ce qu'**il est prévu une délimitation de section transversale (30), laquelle est disposée dans une ramification (28) de la conduite de pression parallèlement à la conduite de pression, dans lequel la ramification est fixée en aval de l'échantillon à tester dans la direction d'écoulement, et la conduite de pression peut être fermée à l'aide d'une soupape d'arrêt (32) en aval de la ramification.

2. Dispositif d'essai selon la revendication 1, **caractérisé en ce qu'**une soupape d'admission (13) et une soupape de décharge (33) sont installées dans la conduite de pression (12).

3. Dispositif d'essai selon l'une au moins des revendications 1 ou 2, **caractérisé en ce qu'**au moins un transmetteur de pression (23, 24) est installé dans la conduite de pression (12), lequel détecte la pression régnant dans la conduite de pression (12).

4. Dispositif d'essai selon la revendication 1 à 3, **caractérisé en ce qu'**au moins deux transmetteurs de pression (23, 24) détectant différentes pressions sont installés dans la conduite de pression (12), en amont desquels est respectivement montée une soupape d'arrêt (21, 22).

5. Dispositif d'essai selon la revendication 4, **caractérisé en ce qu'**une soupape d'arrêt (25) est disposée en aval du transmetteur de pression (24) détectant la pression plus basse.

6. Dispositif d'essai selon l'une au moins des revendications précédentes, **caractérisé en ce que** le dispositif d'essai présente une commande (10).

7. Dispositif d'essai selon l'une au moins des revendications 1 à 6, **caractérisé en ce que** le dispositif d'essai présente un écran (3).

8. Dispositif d'essai selon l'une des revendications 1 à 7, comprenant un moyen d'essai, présentant un corps de base cylindrique (37) doté d'un alésage borgne centrique (38) et une section d'essai (44) avec des alésages (39) radiaux atteignant la surface du corps de base et avec un joint d'étanchéité (43) qui est tendu sur la section d'essai (44), **caractérisé en ce que** le joint d'étanchéité (43) est bloqué par une douille (46) dotée d'une bague torique ainsi que par une bague coulissante (49), lesquels sont respectivement bloquées par un écrou fileté (48, 50).

9. Procédé d'essai d'un échantillon à l'aide d'un dispositif d'essai selon l'une des revendications 1 à 8 avec un échantillon à tester, comprenant les étapes suivantes :
ouverture de la soupape d'admission et de la soupape de décharge (13, 33) remplissage de la conduite de pression (12) avec un fluide fermeture de la soupape d'admission et de la soupape de décharge (13, 33) activation de l'entraînement (34)
commande de l'entraînement (34) selon un profil prédéfini, en particulier selon un profil de pression, un profil de force ou un profil de vitesse de rotation,
détection de la chute de pression.

10. Procédé selon la revendication 9, **caractérisé en ce que** la courbe de pression est enregistrée pendant l'essai.

11. Procédé selon la revendication 9 ou 10, **caractérisé en ce que** la courbe de pression est affichée pendant l'essai.
